# FASCICULE DE BREVET EUROPEEN

(11) **EP 3 160 440 B1**
(45) Date de publication et mention de la délivrance du brevet: **27.10.2021**
(21) Numéro de dépôt: 15732712.3
(22) Date de dépôt: 30.06.2015
(51) Int. Cl.: A61K 8/60, A61K 8/73, A61Q 19/00, A61Q 19/08, A61L 27/00, A61K 8/68

(54) **PRODUITS DE COMBINAISON ET COMPOSITIONS COSMÉTIQUES POUR LUTTER CONTRE LES DÉSORDRES DE LA PEAU ET SON VIEILLISSEMENT AFFECTANT LES KÉRATINOCYTES ET/OU LES FIBROBLASTES ET LE DERME**
KOMBINATIONSPRODUKTE UND KOSMETISCHE ZUSAMMENSETZUNGEN ZUR BEKÄMPFUNG VON HAUTERKRANKUNGEN UND HAUTALTERUNG MIT WIRKUNG AUF KERATINOZYTEN UND/ODER FIBROBLASTEN UND DIE DERMIS
COMBINATION PRODUCTS AND COSMETIC COMPOSITIONS FOR CONTROLLING SKIN DISORDERS AND SKIN AGING THAT AFFECT KERATINOCYTES AND/OR FIBROBLASTS AND THE DERMIS

(30) Priorité: 30.06.2014 FR 1456161; 30.06.2014 FR 1456146
(43) Date de publication de la demande: 03.05.2017
(73) Titulaire: NUTRICOS Technologies, 92117 Clichy Cedex (FR)
(72) Inventeur: MAHE, Yann, F-91700 Ste Genevieve des Bois (FR); BRU, Carole, 25160 Malbuisson (FR)
(74) Mandataire: Lavoix
(86) Numéro de dépôt international: PCT/EP2015/064863
(87) Numéro de publication internationale: WO 2016/001233

(56) Documents cités:
- EP-A1- 1 384 482
- EP-A1- 1 932 530
- EP-A1- 2 033 689
- EP-A2- 1 075 836
- WO-A1-2008/113819
- WO-A2-2012/120290
- CN-A- 101 690 727
- FR-A1- 2 867 073
- US-A- 5 391 373
- US-A1- 2003 068 297
- US-A1- 2003 152 642
- US-A1- 2006 110 459
- US-A1- 2007 092 469
- US-A1- 2008 063 677
- US-A1- 2010 323 984
- US-A1- 2012 141 611
- US-A1- 2013 137 854
- US-A1- 2013 165 845
- US-A1- 2014 005 140
- US-B1- 6 979 458
- KALMAN DOUGLAS S ET AL: "Effect of a natural extract of chicken combs with a high content of hyaluronic acid (Hyal-JointÂ ) on pain relief and quality of life in subjects with knee osteoarthritis: a pilot randomized double-blind placebo-controlled trial", NUTRITION JOURNAL, BIOMED CENTRAL, GB, vol. 7, no. 1, 21 January 2008 (2008-01-21), page 3, XP021036683, ISSN: 1475-2891
- SWANSON HEALTH PRODUCTS: "All-in-one nutrient formula dietary supplement", GNPD, 1 November 2011 (2011-11-01), XP002741682,
- DATABASE GNPD [Online] MINTEL; 4 June 2010 (2010-06-04), anonymous: "Arthrimin GS Glucosamine Chondroitin Effervescent Drink", XP055645795, retrieved from www.gnpd.com Database accession no. 1333413
- DATABASE GNPD [Online] MINTEL; 13 October 2010 (2010-10-13), anonymous: "Bone & Joint Formula 7 Tablets", XP055645793, retrieved from www.gnpd.com Database accession no. 1416187
- DATABASE GNPD [Online] MINTEL; 3 August 2010 (2010-08-03), anonymous: "Extra Strength Joint Care Caplets", XP055646306, retrieved from www.gnpd.com Database accession no. 1369680

## Description

La présente invention concerne l'utilisation cosmétique non thérapeutique d'un produit de combinaison cosmétique pour une administration simultanée, séparée ou étalée dans le temps, comprenant : une première composition cosmétique comprenant du sulfate de glucosamine, une deuxième composition cosmétique comprenant de l'acide hyaluronique, et une troisième composition cosmétique comprenant du collagène, pour induire la synthèse d'acide hyaluronique par les fibroblastes, et ainsi lutter contre le vieillissement de la peau affectant les fibroblastes et le derme, ainsi qu'un procédé cosmétique mettant en œuvre ces produits de combinaison.

L'acide hyaluronique est une macromolécule constituée d'un enchainement répétitif d'un motif sucré qui présente des propriétés importantes d'hydratation et de soutien des tissus. La peau qui contient environ 50% de l'acide hyaluronique du corps humain est un des organes les plus riches en acide hyaluronique. Le renouvellement de l'acide hyaluronique dans la peau est rapide, quasi quotidien. Avec le vieillissement, son renouvellement est altéré.

Il existe donc un besoin d'aide à l'organisme pour renouveler la quantité d'acide hyaluronique, notamment afin d'éviter les affaissements du tissu matriciel sous cutané, l'apparition des rides ou encore la perte d'hydratation de la peau.

Ainsi, de nombreux produits cosmétiques proposent des crèmes contenant de l'acide hyaluronique pour aider à maintenir une hydratation de la peau et lutter contre son vieillissement.

Néanmoins, les tentatives de réintroduction d'acide hyaluronique par voie orale se sont souvent révélées infructueuses. En effet, cette grosse molécule polysaccharidique ne passe pas la barrière intestinale avec efficacité. De plus, compte tenu de sa nature biochimique, elle est rapidement dégradée.

Pour contourner ce problème, une technique esthétique très utilisée consiste à repulper directement les rides ou certaines zones cutanées en les remplissant avec des polymères d'acide hyaluronique au moyen d'une seringue (le terme de « fillers » est employé).

Cette technique présente cependant l'inconvénient de devoir être renouvelée tous les six mois environ car l'acide hyaluronique nouvellement injecté se dégrade aussi au cours du temps.Le problème technique à la base de la présente invention consiste donc à pouvoir stimuler la synthèse endogène d'acide hyaluronique et ainsi prolonger sa synthèse dans la peau, notamment par voie orale, par exemple en complément des interventions topiques ou des injections esthétiques déjà existantes.

La glucosamine est couramment utilisée, notamment par voie orale, pour lutter contre l'ostéoarthrose. En effet, la glucosamine est un des deux constituants naturels de l'acide hyaluronique, celui-ci correspondant à un enchainement polymérique de dimères disaccharidiques constitués de N-Acétyl glucosamine d'une part et d'acide D-glucuronique d'autre part, liés en eux par une liaison saccharidique.

US 2003/152642 décrit un complément alimentaire sous forme de snack ou de boisson comprenant de la glucosamine et de l'acide hyaluronique pour traiter les dysfonctionnements du cartilage.

La glucosamine, contrairement aux grosses molécules d'acide hyaluronique, est très rapidement biodisponible et se retrouve dans la circulation sanguine rapidement après ingestion. Elle y persiste même pendant plusieurs heures (Persiani S. et al. Glucosamine oral bioavailability and plasma pharmacokinetics after increasing doses of crystalline glucosamine sulfate in man. OsteoArthtitis and Cartilage. 2005; 13: 1041-1049).

La glucosamine pourrait ainsi être utile en tant que précurseur par voie orale de la synthèse endogène d'acide hyaluronique pour la santé articulaire. Elle est en effet connue pour induire la synthèse d'acide hyaluronique, notamment par des implants de cellules osseuses *in vitro* (Ultterlinden et al. Glucosamine increases hyaluronic acid production in human osteoarthritic synovium explants. BMC musculoskeletal disorders 2008,9:120-125).

Pour répondre aux préoccupations liées plus spécifiquement à la synthèse d'acide hyaluronique dans la peau, les inventeurs de la présente invention ont montré de façon très surprenante et inattendue qu'une association entre la glucosamine ou l'un de ses sels et de l'acide hyaluronique était capable d'induire la synthèse d'acide hyaluronique par des cellules cutanées : les kératinocytes, alors qu'une association entre la glucosamine ou l'un de ses sels et de l'acide hyaluronique était capable d'induire la synthèse d'acide hyaluronique par des cellules du derme : les fibroblastes, mais en présence de collagène.

En effet, si les inventeurs de la présente demande ont observé que l'utilisation de la glucosamine ou l'un de sels permettait d'induire la synthèse d'acide hyaluronique par les kératinocytes, ils ont cependant constaté que les fibroblastes humains étaient réfractaires à cet effet. De plus, ils ont également constaté que le contact des kératinocytes avec de l'acide hyaluronique seule ne stimulait pas la propre synthèse de celui-ci et que de l'acide hyaluronique seul ou un extrait de cartilage comprenant de l'acide hyaluronique et du collagène, ne stimulait pas la synthèse d'acide hyaluronique, par le contact avec des fibroblastes.

Ainsi, les inventeurs de la présente invention ont mis en évidence de façon très surprenante que seule l'association de la glucosamine ou l'un de ses sels avec de l'acide hyaluronique stimulait de façon synergique et très importante la synthèse d'acide hyaluronique par les kératinocytes et que seule l'association de la glucosamine ou l'un de ses sels avec de l'acide hyaluronique et du collagène stimulait de façon synergique et très importante la synthèse d'acide hyaluronique par les fibroblastes.

Sur la base des effets obtenus, ces combinaisons synergiques permettent donc de lutter respectivement contre le vieillissement de la peau affectant les kératinocytes, en particulier contre les signes du vieillissement liés à la perte de tissu matriciel, et contre le vieillissement de la peau affectant les fibroblastes, en particulier contre les signes du vieillissement liés à la perte de tissu conjonctif et dermique.

La présente invention est telle que définie dans l'objet des revendications 1 à 24.

Elle concerne l'utilisation cosmétique non thérapeutique d'un produit de combinaison cosmétique pour une administration simultanée, séparée ou étalée dans le temps, comprenant une première composition cosmétique comprenant du sulfate de glucosamine, une deuxième composition cosmétique comprenant de l'acide hyaluronique, et une troisième composition cosmétique comprenant du collagène pour induire la synthèse d'acide hyaluronique par les fibroblastes.

Par « produit de combinaison cosmétique » ou « composition cosmétique », on entend une substance ou une préparation destinée à être mise en contact avec les diverses parties superficielles du corps humain, notamment l'épiderme, les systèmes pileux et capillaire, les ongles, les lèvres et les organes génitaux externes, ou avec les dents et les muqueuses buccales, en vue, exclusivement ou principalement, de les nettoyer, de les embellir, de les parfumer, d'en modifier l'aspect, de les protéger, de les maintenir en bon état ou de corriger les odeurs corporelles. On entend également, par exemple, une composition nutritionnelle, par exemple un complément alimentaire.

Par « complément alimentaire », on entend désigner ici une denrée alimentaire dont le but est de compléter le régime alimentaire normal et qui constitue une source concentrée de nutriments, c'est-à-dire vitamines et/ou minéraux, et/ou d'autres substances ayant un effet nutritionnel ou physiologique, seuls ou combinés, commercialisée sous forme de doses, à savoir les formes de présentation telles que les gélules, les pastilles, les comprimés, les capsules molles, les pilules et autres formes similaires, ainsi que les sachets de poudre à diluer, les ampoules de liquide, les flacons munis d'un compte-gouttes et les autres formes analogues de préparations liquides ou sous forme de boissons.

La présente invention ne se rapporte pas au domaine thérapeutique.

Par « sels » de la glucosamine, on entend tout sel de la glucosamine pouvant être formulé dans une composition cosmétique tel que par exemple la glucosamine sulfate ou la glucosamine hydrochloride. Le sel de glucosamine compris dans les produits de combinaison cosmétiques selon l'invention est le sulfate de glucosamine.

L'acide hyaluronique compris dans les produits de combinaison cosmétiques selon l'invention peut être de diverses origines. Il peut par exemple être obtenu par voie biotechnologique telle qu'issu d'une synthèse bactérienne. En particulier, l'acide hyaluronique compris dans les produits de combinaison cosmétiques selon l'invention est d'origine bactérienne. Dans certains modes de réalisation, l'acide hyaluronique peut être compris dans tout élément en comprenant ou permettant d'en obtenir, comme par exemple du cartilage issu d'une source alimentaire issue de poisson ou de mammifère comme le porc, le bœuf ou la volaille, et plus préférentiellement issu du poulet.

Le collagène compris dans les produits de combinaison cosmétiques selon l'invention peut être de diverses origines. Il peut être compris dans tout élément en comprenant ou permettant d'en obtenir, comme par exemple du cartilage, préférentiellement un cartilage issu d'une source alimentaire issue de poisson ou de mammifère comme le porc, le bœuf ou la volaille, et plus préférentiellement issu du poulet. Le collagène peut également être issu d'un procédé de production biotechnologique et obtenu notamment à partir de souches bactériennes.

Selon un mode de réalisation des produits de combinaison cosmétiques selon l'invention comprenant du sulfate de glucosamine, de l'acide hyaluronique et du collagène, l'acide hyaluronique et le collagène sont compris dans une même composition cosmétique et sont issus d'un extrait de cartilage en particulier de poulet.

En particulier, les produits de combinaison cosmétiques selon l'invention comprennent de 0.0001 à 99 % de sulfate de glucosamine, en particulier de 0.001 à 50 %, et plus particulièrement encore de 0.1 à 10%.

En particulier également, les produits de combinaison cosmétiques comprenant du sulfate de glucosamine, de l'acide hyaluronique et du collagène, comprennent au moins 0.1% d'acide hyaluronique, en particulier au moins 2% et plus particulièrement encore au moins 4%, en poids, par rapport au poids total du produit de combinaison cosmétique.

En particulier également, les produits de combinaison cosmétiques selon l'invention, comprennent de 0.1 à 99% d'acide hyaluronique, en particulier de 10 à 30% et plus particulièrement encore de 16 à 25%.

Plus particulièrement, les produits de combinaison cosmétiques selon l'invention, , comprennent au moins 0.1% de collagène, de préférence 0.1% à 99% de collagène, en particulier de 1 à 60%, et plus particulièrement encore de 1 à 10% en poids, par rapport au poids total du produit de combinaison cosmétique .

Selon un mode de réalisation de la présente invention, les compositions cosmétiques des produits de combinaison cosmétiques selon l'invention, sont destinées à être administrées par voie orale.

Selon un autre mode de réalisation de la présente invention, les compositions cosmétiques des produits de combinaison cosmétiques selon l'invention, sont destinées à être administrées par voie topique.

Selon encore un autre mode de réalisation de la présente invention, dans les produits de combinaison cosmétiques selon l'invention, la composition cosmétique comprenant du sulfate de glucosamine est destinée à être administrée par voie orale et la ou les composition(s) cosmétique(s) comprenant de l'acide hyaluronique et du collagène est(sont) destinée(s) à être administrée(s) par voie topique.

Selon encore un autre mode de réalisation de la présente invention, dans les produits de combinaison cosmétiques selon l'invention, les compositions comprenant l'acide hyaluronique et le collagène sont destinées à être administrées par la voie orale et la composition comprenant le sulfate de glucosamine est destinée à être administrée par la voie topique.

Selon un mode de réalisation de la présente invention, dans les produits de combinaison cosmétiques selon l'invention comprenant des compositions cosmétiques comprenant du sulfate de glucosamine, de l'acide hyaluronique et du collagène, les compositions comprenant du sulfate de glucosamine et le collagène sont destinées à être administrées par la voie orale et la composition comprenant l'acide hyaluronique est destinée à être administrée par la voie topique.

Selon un mode de réalisation distinct de la présente invention, dans les produits de combinaison cosmétiques selon l'invention, la ou les composition(s) cosmétique(s) comprenant du sulfate de glucosamine et le collagène est(sont) destinée(s) à être administrée(s) par voie orale et la composition cosmétique comprenant de l'acide hyaluronique est une composition cosmétique de comblement (filler) destinée à être administrée dans la peau, notamment dans le cadre de la préparation des fillers à l'acide hyaluronique.

Par « composition de comblement » ou « filler », on entend une composition destinée à combler les rides (rides et sillons permanents), se présentant notamment sous la forme de gel.

Ainsi, que les compositions cosmétiques du produit de combinaison selon l'invention soient toutes administrées par la voie orale, toutes administrées par la voie topique, ou administrées selon des modes d'administration différents, comme exemplifié ci-dessus, celles-ci pourront indépendamment l'une de l'autre être administrées de façon simultanée, de façon séparée ou de façon étalée dans le temps.

Selon un mode de réalisation de la présente invention, le produit de combinaison cosmétique ne comprend pas d'autres actifs que ceux listés ci-dessus.

Pour une administration par voie orale, les compositions cosmétiques selon l'inventionfaisant partie des produits de combinaison cosmétiques selon l'invention, peuvent se présenter sous toute forme adaptée, et prendre la forme d'une dragée, d'une gélule, d'une suspension, d'un gel, d'une émulsion, d'une solution buvable, d'un comprimé à avaler ou à croquer, d'une capsule, notamment d'une capsule molle ou dure, d'un granulé à dissoudre, d'un sirop, d'une tablette ou d'une ampoule buvable.

Elle peut de préférence se présenter sous la forme d'une capsule, molle ou dure, de préférence sous forme d'une capsule molle.

En particulier, une composition conforme à l'invention peut être mise en œuvre dans toutes formes de compléments alimentaires ou des poudres compactées ou non. Les poudres peuvent être diluées à l'eau ou dans du soda. Selon un mode de réalisation, une composition conforme à l'invention administrée par voie orale peut être formulée sous forme de dragée, de gélule, de gel, d'émulsion, de comprimé, de capsule, d'hydrogel, de poudre compactée ou non, de suspension ou solution liquide.

Les compositions par voie orale peuvent se présenter soit sous forme anhydre, soit sous forme aqueuse.

Une composition conforme à l'invention peut être formulée avec les excipients usuels pour de telles compositions orales, tels que des compléments alimentaires, à savoir notamment des composants gras et/ou aqueux, des agents humectants, épaississants, conservateurs, des agents de texture, de saveur et/ou d'enrobage, des antioxydants, des conservateurs et des colorants usuels dans le domaine de l'alimentaire.

Dans le cas de compositions convenant à une administration par voie orale, on privilégie l'utilisation d'un support ingérable. Le support ingérable peut être de nature diverse selon le type de composition considérée.

La formulation de telles compositions peut être réalisée par tout procédé usuel connu de l'homme du métier.

Pour une application topique sur la peau, les compositions cosmétiques selon l'invention peuvent avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses. Ces compositions sont préparées selon les méthodes usuelles.

Ces compositions cosmétiques peuvent par exemple constituer des crèmes de nettoyage/démaquillage, de maquillage, de protection, de traitement ou de soin pour le visage (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage.

Les compositions peuvent aussi être conditionnées sous forme de composition pour aérosol comprenant également un agent propulseur sous pression.

Les compositions cosmétiques selon l'invention peuvent classiquement contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Bien entendu l'homme du métier veillera à choisir les éventuels excipients et agents de formulation et/ou leur quantité de telle manière que les propriétés avantageuses du produit de combinaison cosmétique selon l'invention ou de la composition cosmétique selon l'invention ne soient pas ou substantiellement pas altérées par l'adjonction envisagée.

A titre d exemple, un produit de combinaison cosmétique selon l'invention présente la composition suivante :

### Produit comprimé pour voie orale :

Glucosamine sulfate 125 mg
Acide hyaluronique 60 mg
Collagène 5 mg
Excipients QSP 380 mg

Comme préalablement mentionné, de façon tout à fait surprenante et inattendue, les inventeurs de la présente invention ont mis en évidence que les produits de combinaison cosmétiques selon l'invention permettent d'induire la synthèse d'acide hyaluronique.

Plus particulièrement, lesdits produits de combinaison sont utiles pour induire la synthèse cutanée ou épidermique d'acide hyaluronique, en particulier au niveau du derme et/ou de la jonction dermo épidermique.

Ainsi, la présente invention concerne l'utilisation cosmétique d'un produit de combinaison cosmétique tel que défini dans le cadre de la présente invention, pour induire la synthèse d'acide hyaluronique, en particulier pour induire la synthèse cutanée ou épidermique d'acide hyaluronique, plus particulièrement au niveau du derme et/ou de la jonction dermo épidermique.

La synthèse d'acide hyaluronique est induite par les fibroblastes du derme cutané.

Ainsi, la présente invention concerne également l'utilisation d'un produit de combinaison cosmétique selon l'invention pour renforcer la jonction dermo épidermique et/ou la synthèse d'acide hyaluronique par le derme et le tissu conjonctif de la jonction dermo-épidermique.

En particulier, le produit de composition cosmétique selon l'invention est utile pour renforcer la jonction dermo épidermique et/ou la synthèse d'acide hyaluronique par le derme et le tissu conjonctif de la jonction dermo-épidermique.

En particulier, les produits de combinaison cosmétiques selon l'invention permettent de lutter contre le vieillissement de la peau affectant les fibroblastes.

Plus particulièrement, les produits de combinaison cosmétiques selon l'invention permettent de lutter contre les signes du vieillissement liés à la perte de tissu matriciel, à la sécheresse et à l'aspect papyracé de la peau.

En particulier, les produits de combinaison cosmétiques selon l'invention permettent de lutter contre les signes du vieillissement liés à la perte de tissu matriciel, à l'apparition des rides et ridules et à l'affaissement de la peau.

En particulier, les produits de combinaison cosmétiques selon l'invention permettent de lutter contre les signes du vieillissement liés à la perte de tissu conjonctif et dermique.

En particulier, les produits de combinaison cosmétiques selon l'invention permettent de lutter contre le vieillissement de la peau affectant l'épiderme, en particulier pour lutter contre les désordres d'hydratation et de desquamation de l'épiderme, notamment ceux aggravés par une exposition solaire intense,.

Ainsi, lesdits produits sont utiles pour améliorer les effets et la durée des traitements cosmétiques à base d'acide hyaluronique, comme par exemple les traitements topiques, les traitements utilisant des compositions de comblement (« fillers »), les traitements utilisant des patchs, etc.

Ils sont par ailleurs également utiles pour renforcer l'hydratation de la peau.

Dans le cadre de la présente invention, les produits de combinaison cosmétiques selon l'invention sont utilisés pour la préparation de peaux reconstruites et d'implants cutanés.

Par conséquent, la présente invention concerne également l'utilisation d'un produit de combinaison cosmétique selon l'invention pour lutter contre le vieillissement de la peau affectant les fibroblastes, en particulier contre les signes du vieillissement liés à la perte de tissu matriciel ou de tissu conjonctif et dermique et/ou pour améliorer les effets des traitements cosmétiques à base d'acide hyaluronique et/ou pour renforcer l'hydratation de la peau et/ou pour la préparation de peaux reconstruites et d'implants cutanés.

Les produits de combinaison cosmétiques selon l'invention peuvent également être mis en œuvre dans un procédé cosmétique destiné à induire la synthèse d'acide hyaluronique.

Ainsi, selon l'un de ses aspects, la présente invention concerne également un procédé cosmétique pour induire la synthèse d'acide hyaluronique, dans lequel un produit de combinaison cosmétique tel que défini dans le cadre de la présente invention, est administré(e), ledit procédé induisant la synthèse d'acide hyaluronique par les fibroblastes.

En particulier, la présente invention concerne un procédé cosmétique tel que mentionné ci-dessus pour induire la synthèse épidermique d'acide hyaluronique, en particulier au niveau de la jonction dermo épidermique.

Ainsi, selon un de ces aspects, la présente invention concerne un procédé cosmétique tel que précédemment décrit, pour renforcer la jonction dermo épidermique et/ou la synthèse de tissu conjonctif de la jonction dermo-épidermique et/ou pour lutter contre le vieillissement de la peau affectant les fibroblastes, en particulier contre les signes du vieillissement liés à la perte de tissu matriciel ou de tissu conjonctif et dermique et/ou pour améliorer les effets des traitements cosmétiques à base d'acide hyaluronique et/ou pour renforcer l'hydratation de la peau et/ou pour préparer des peaux reconstruites et des implants cutanés.

Tout particulièrement, les produits de combinaison cosmétiques s selon l'invention, peuvent également être mis en œuvre dans un procédé cosmétique destiné à limiter l'apparition des rides, des ridules et l'affaissement du visage, notamment pour lutter contre la réapparition des rides et l'affaissement du visage après des injections d'acide hyaluronique dans la peau, sur le visage et/ou sur l'ensemble des parties du corps ayant fait l'objet d'une procédure d'injection réparatrice, esthétique ou cosmétique ultérieure ou concomitante, sans toutefois dépasser 6 mois entre les 2 procédures.

Ainsi, selon l'un de ses aspects, la présente invention concerne également un procédé cosmétique pour limiter l'apparition des rides, des ridules et l'affaissement du visage, notamment pour lutter contre la réapparition des rides et l'affaissement du visage après des injections d'acide hyaluronique dans la peau, sur le visage et/ou sur l'ensemble des parties du corps ayant fait l'objet d'une procédure d'injection réparatrice, esthétique ou cosmétique ultérieure ou concomitante, sans toutefois dépasser 6 mois entre les 2 procédures, dans lequel un produit de combinaison cosmétique tel que défini dans le cadre de la présente invention, est administré.

Les produits de combinaison cosmétiques selon l'invention, peuvent également être mis en œuvre dans un procédé cosmétique destiné à prolonger la durée d'efficacité des traitements esthétiques réalisés au moyen de compositions de comblement (filler), notamment à base d'acide hyaluronique injecté dans la peau pour le comblement du visage, du corps et des rides.

Ainsi, selon un autre de ses aspects, la présente invention concerne également un procédé cosmétique pour prolonger la durée d'efficacité des traitements esthétiques réalisés au moyen de compositions de comblement (filler), notamment à base d'acide hyaluronique injecté dans la peau pour le comblement du visage, du corps et des rides, dans lequel un produit de combinaison cosmétique tel que défini dans le cadre de la présente invention, est administré.

Selon un mode de mise en œuvre du procédé cosmétique selon l'invention, les compositions cosmétiques du produit de combinaison tel que précédemment décrit sont administrées par voie orale.

Selon un autre mode de mise en œuvre du procédé cosmétique selon l'invention, les compositions cosmétiques du produit de combinaison tel que précédemment décrit sont administrées par voie topique.

Selon encore un autre mode de mise en œuvre du procédé cosmétique selon l'invention, dans le produit de combinaison cosmétique tel que précédemment décrit, la composition comprenant le sulfate de glucosamine est administrée par voie orale et la ou les composition(s) comprenant de l'acide hyaluronique et le collagène est(sont) administrée(s) par voie topique.

Selon encore un autre mode de mise en œuvre du procédé cosmétique selon l'invention, dans les produits de combinaison cosmétiques selon l'invention, la ou les composition(s) comprenant l'acide hyaluronique et le collagène est(sont) destinée(s) à être administrée(s) par la voie orale et la composition comprenant le sulfate de glucosamine est destinée à être administrée par la voie topique.

Selon un mode de mise en œuvre distinct du procédé cosmétique selon l'invention, dans le produit de combinaison cosmétique selon l'invention, la composition comprenant le sulfate de glucosamine est administrée par voie orale et la ou les composition(s) comprenant de l'acide hyaluronique et le collagène est (sont) une composition cosmétique de comblement (filler) administrée dans la peau.

Selon un mode de mise en œuvre du procédé cosmétique selon l'invention, dans les produits de combinaison cosmétique selon l'invention, la ou les composition(s) comprenant le sulfate de glucosamine et le collagène est(sont) destinée(s) à être administrée(s) par la voie orale et la composition comprenant l'acide hyaluronique est destinée à être administrée par la voie topique.

Selon un autre mode de de mise en œuvre du procédé cosmétique selon l'invention, dans les produits de combinaison cosmétique selon l'invention, la ou les composition(s) comprenant le sulfate de glucosamine et le collagène est(sont) destinée(s) à être administrée(s) par la voie orale et la composition comprenant l'acide hyaluronique est une composition cosmétique de comblement (filler) destinée à être administrée la peau, notamment dans le cadre de la préparation des fillers à l'acide hyaluronique.

Dans la description, les plages de valeurs libellées sous la forme « entre ... et ... » incluent les bornes inférieure et supérieure précisées.

D'autres caractéristiques et avantages de l'invention ressortiront des exemples qui suivent, donnés à titre illustratif et non limitatif.

### FIGURES

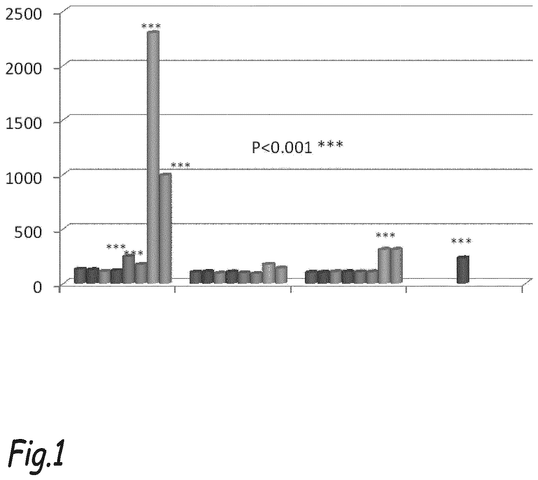

**Figure 1** : Effet synergique du sulfate de glucosamine et de l'acide hyaluronique sur la production d'acide hyaluronique par des cellules NHEK (Normal Human Epidermal Keratinocytes) (ne fait pas partie de l'invention): quatre séries de résultats sont indiquées (de gauche à droite) qui représentent :
   - 1ère série : production d'acide hyaluronique par des cellules NHEK obtenue au moyen de la combinaison entre le sulfate de glucosamine et l'acide hyaluronique dans les proportions suivantes de sulfate de glucosamine (µg/ml)-acide hyaluronique (ng/ml) (de gauche à droite) : 0.25à 25 µg/ml - 40 à 6000 ng/ml;
   - 2^{ème} série : production d'acide hyaluronique par des cellules NHEK obtenue au moyen de l'acide hyaluronique seul dans les proportions suivantes d'acide hyaluronique (ng/ml) (de gauche à droite) : 40 à 6000 ng/ml;
   - 3^{ème} série : production d'acide hyaluronique par des cellules NHEK obtenue au moyen du sulfate de glucosamine seul dans les proportions suivantes de sulfate de glucosamine (µg/ml) (de gauche à droite) : 0.25 à 25 µg/ml ; et
   - 4^{ème} série : contrôle RA.

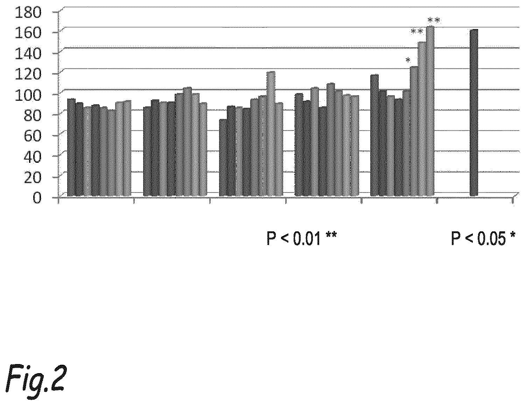

**Figure 2** : Effet synergique du sulfate de glucosamine, de l'acide hyaluronique et du collagène sur la production d'acide hyaluronique par des cellules NHDF (Normal Human Dermal Fibroblasts) : six séries de résultats sont indiquées (de gauche à droite) qui représentent :
   - 1^{ère} série : production d'acide hyaluronique par des cellules NHDF obtenue au moyen du sulfate de glucosamine seul dans les proportions suivantes de sulfate de glucosamine (µg/ml) (de gauche à droite) : 0.1 à 100 µg/ml;
   - 2^{ème} série : production d'acide hyaluronique par des cellules NHDF obtenue au moyen de l'acide hyaluronique seul dans les proportions suivantes d'acide hyaluronique (ng/ml) (de gauche à droite) : 40 à 6000 ng/ml;
   - 3^{ème} série : production d'acide hyaluronique par des cellules NHDF obtenue au moyen de la combinaison du sulfate de glucosamine et de l'acide hyaluronique dans les proportions suivantes de sulfate de glucosamine (µg/ml) et d'acide hyaluronique (ng/ml) (de gauche à droite) : 0.1 à 100 µg/ml et 40 à 6000 ng/ml;
   - 4^{ème} série : production d'acide hyaluronique par des cellules NHDF obtenue au moyen de la combinaison d'une source d'acide hyaluronique et de collagène seule dans les proportions suivantes (ng/ml) : de 4 à 4000 ng/ml ;
   - 5^{ème} série : production d'acide hyaluronique par des cellules NHDF obtenue au moyen de la combinaison de sulfate de glucosamine et d'une source d'acide hyaluronique et de collagène dans les proportions suivantes de sulfate de glucosamine (µg/ml) et de source d'acide hyaluronique et de collagène (ng/ml) : 0.1 à 100 µg/ml et 4 à 4000 ng/ml; et
   - 6^{ème} série : contrôle TGF-beta.

### EXEMPLES

### EXEMPLE 1: Effet synergique du sulfate de glucosamine et de l'acide hyaluronique sur la production d'acide hyaluronique par des cellules NHEK (ne fait pas partie de l'invention).

Des kératinocytes épidermiques humains normaux sont mis en culture à 37°C sous 5% de CO₂ dans un milieu de culture kératinocyte-SFM supplémenté avec du facteur de croissance épidermique (EGF ; 0.25ng/ml), un extrait pituitaire (25µg/ml) et un antibiotique (Gentamicine 25µg/ml) dans des plaques de 96 puits pendant 24 heures sans stimulation.

La glucosamine et/ou l'acide hyaluronique sont ensuite ajoutés au milieu de culture seuls ou en association à des concentrations allant de 40 à 6000ng/ml d'acide hyaluronique et de 0.25 à 25 µg/ml pour la glucosamine sulfate. Après 72 heures de culture dans ces conditions, les surnageant sont collectés afin de quantifier l'acide hyaluronique produit par les cellules au moyen d'un kit ELISA Duoset Hyaluronan commercialisé par la société R&D system (référence DY3614).

Les résultats obtenus et présentés dans la Figure 1 montrent une induction de la production d'acide hyaluronique par les kératinocytes humains *in vitro,* obtenue au moyen du sulfate de glucosamine seul.

Ils démontrent également que le contact des kératinocytes avec de l'acide hyaluronique seul ne stimule pas la synthèse d'acide hyaluronique. Cependant, une induction synergique de la production d'acide hyaluronique par les kératinocytes humains *in vitro,* est clairement obtenue en associant le sulfate de glucosamine et de l'acide hyaluronique.

En effet, les résultats observés permettent ici de conclure à un effet synergique entre le sulfate de glucosamine et l'acide hyaluronique en comparaison avec les résultats obtenus avec chacun des composés seuls, avec un effet augmenté de 246% (p<0.001).

### EXEMPLE 2 : Effet synergique du sulfate de glucosamine, de l'acide hyaluronique et du collagène sur la synthèse d'acide hyaluronique

Des fibroblastes dermiques humains normaux (NHDF) sont mis en culture à 37°C sous 5% de CO₂ dans un milieu de culture DMEM supplémenté par de la glutamine (2mM); du sérum de veau (1%), de la penicilline (50U/ml) et de la streptomycine (50µg/ml).

La glucosamine (de 0.1 à 100 µg/ml) et/ou de l'acide hyaluronique (de 40 à 6000 ng/ml) et/ou une source de collagène et d'acide hyaluronique (dans un ratio compris entre 0,07% et 15%) à des concentrations allant de 4 à 4000ng/ml sont ensuite ajoutés au milieu de culture seuls ou en association.

Après 72 heures de culture dans ces conditions les surnageant sont collectés afin de quantifier l'acide hyaluronique produit par les cellules au moyen d'un kit ELISA Duoset Hyaluronan commercialisé par la société R&D system (réf DY3614).

Les résultats obtenus et présentés dans la Figure 2 montrent qu'une induction synergique de la production d'acide hyaluronique par les fibroblastes humains *in vitro,* est clairement obtenue en associant le sulfate de glucosamine, de l'acide hyaluronique et du collagène.

En effet, les résultats observés permettent ici de conclure à un effet synergique entre le sulfate de glucosamine, l'acide hyaluronique et le collagène en comparaison avec l'absence de stimulation obtenue en administrant du sulfate de glucosamine seul ou un extrait naturel de cartilage seul comprenant de l'acide hyaluronique et du collagène ou encore l'association seule d'acide hyaluronique et de glucosamine.

## Revendications

1. Utilisation cosmétique non thérapeutique d'un produit de combinaison cosmétique pour une administration simultanée, séparée ou étalée dans le temps, comprenant : une première composition cosmétique comprenant du sulfate de glucosamine, une deuxième composition cosmétique comprenant de l'acide hyaluronique et une troisième composition cosmétique comprenant du collagène pour induire la synthèse d'acide hyaluronique par les fibroblastes.

2. Utilisation cosmétique non thérapeutique selon la revendication 1, dans laquelle ladite deuxième composition cosmétique comprenant de l'acide hyaluronique et ladite troisième composition cosmétique comprenant le collagène constituent une même composition cosmétique dans laquelle l'acide hyaluronique et le collagène sont issus d'un extrait de cartilage, préférentiellement un cartilage issu d'une source alimentaire issue de poisson ou de mammifère comme le porc, le bœuf ou la volaille, et plus préférentiellement issu du poulet.

3. Utilisation cosmétique non thérapeutique selon la revendication 1, dans laquelle l'acide hyaluronique de la deuxième composition cosmétique est d'origine biotechnologique et de préférence bactérienne.

4. Utilisation cosmétique non thérapeutique selon l'une des revendications 1 à 3, dans laquelle le collagène est issu d'un procédé de purification à partir de poisson, de mammifère ou de volaille.

5. Utilisation cosmétique non thérapeutique selon l'une quelconque des revendications 1 à 4, dans laquelle lesdites compositions sont destinées à être administrées par voie topique.

6. Utilisation cosmétique non thérapeutique selon l'une quelconque des revendications 1 à 4, dans laquelle la composition comprenant du sulfate de glucosamine est destinée à être administrée par voie orale et les compositions comprenant l'acide hyaluronique et/ou le collagène sont destinées à être administrées par voie topique.

7. Utilisation cosmétique non thérapeutique selon l'une quelconque des revendications 1 à 4 dans laquelle la composition comprenant l'acide hyaluronique et/ou le collagène sont destinées à être administrées par la voie orale et la composition comprenant le sulfate de glucosamine est destinée à être administrée par la voie topique.

8. Utilisation cosmétique non thérapeutique selon l'une quelconque des revendications 1 à 4, dans laquelle la composition comprenant du sulfate de glucosamine est destinée à être administrée par voie orale et les compositions comprenant de l'acide hyaluronique et/ou le collagène sont des compositions cosmétiques de comblement (filler) destinées à être administrées dans la peau, notamment dans le cadre de la préparation des fillers à l'acide hyaluronique.

9. Utilisation cosmétique non thérapeutique selon l'une quelconque des revendications 1 à 4, dans laquelle les compositions comprenant du sulfate de glucosamine et/ou le collagène sont destinées à être administrées par la voie orale et la composition comprenant l'acide hyaluronique est destinée à être administrée par la voie topique.

10. Utilisation cosmétique non thérapeutique selon l'une quelconque des revendications 1 à 4, dans laquelle les compositions comprenant du sulfate de glucosamine et/ou le collagène sont destinées à être administrées par la voie orale et la composition comprenant l'acide hyaluronique est une composition cosmétique de comblement (filler) destinée à être administrée dans la peau, notamment dans le cadre de la préparation des fillers à l'acide hyaluronique.

11. Utilisation cosmétique non thérapeutique selon l'une quelconque des revendications 1 à 10, dans laquelle le produit de combinaison comprend au moins 0.0001% d'acide hyaluronique et/ou au moins 0.1% de sulfate de glucosamine et/ou au moins 0.1% de collagène, en poids par rapport au poids total du produit de combinaison cosmétique.

12. Utilisation cosmétique non thérapeutique selon l'une quelconque des revendications 1 à 11, dans laquelle le produit de combinaison comprend de 10 à 30% d'acide hyaluronique en poids par rapport au poids total du produit de combinaison cosmétique.

13. Utilisation cosmétique non thérapeutique selon l'une quelconque des revendications 1 à 12, pour renforcer la jonction dermo épidermique et/ou la synthèse d'acide hyaluronique par le derme et le tissu conjonctif de la jonction dermo-épidermique.

14. Utilisation cosmétique non thérapeutique selon l'une quelconque des revendications 1 à 13 pour lutter contre les signes du vieillissement liés à la perte de tissu conjonctif et dermique.

15. Utilisation cosmétique non thérapeutique selon l'une quelconque des revendications 1 à 13 pour lutter contre les signes du vieillissement liés à la perte de tissu matriciel, à la sécheresse et à l'aspect papyracé de la peau.

16. Utilisation cosmétique non thérapeutique selon l'une quelconque des revendications 1 à 13, pour lutter contre les signes du vieillissement liés à la perte de tissu matriciel, à l'apparition des rides et ridules et à l'affaissement de la peau.

17. Utilisation cosmétique non thérapeutique selon l'une quelconque des revendications 1 à 13, pour lutter contre le vieillissement de la peau affectant l'épiderme, en particulier pour lutter contre les désordres d'hydratation et de desquamation de l'épiderme, notamment ceux aggravés par une exposition solaire intense.

18. Utilisation cosmétique non thérapeutique selon l'une quelconque des revendications 1 à 13, pour améliorer les effets et la durée des traitements cosmétiques à base d'acide hyaluronique, en particulier l'effet des compositions de comblement (filler) comprenant de l'acide hyaluronique.

19. Utilisation cosmétique non thérapeutique selon l'une quelconque des revendications 1 à 13, pour renforcer l'hydratation de la peau.

20. Utilisation cosmétique non thérapeutique selon l'une quelconque des revendications 1 à 13, pour la préparation de peaux reconstruites et d'implants cutanés.

21. Procédé cosmétique non thérapeutique pour induire la synthèse d'acide hyaluronique par les fibroblastes, dans lequel un produit de combinaison cosmétique tel que défini selon l'une quelconque des revendications 1 à 12 est administré(e).

22. Procédé cosmétique non thérapeutique selon la revendication 21 pour limiter l'apparition des rides, des ridules et l'affaissement du visage.

23. Procédé cosmétique non thérapeutique selon la revendication 21, pour lutter contre la réapparition des rides et l'affaissement du visage après des injections d'acide hyaluronique dans la peau, sur le visage et/ou sur l'ensemble des parties du corps ayant fait l'objet d'une procédure d'injection réparatrice, esthétique ou cosmétique ultérieure ou concomitante, sans toutefois dépasser 6 mois entre les 2 procédures.

24. Procédé cosmétique non thérapeutique selon la revendication 21 pour prolonger la durée d'efficacité des traitements esthétiques réalisés au moyen de compositions de comblement (filler), notamment à base d'acide hyaluronique injecté dans la peau pour le comblement du visage, du corps et des rides.

## Patentansprüche

1. Nicht-therapeutische kosmetische Verwendung eines kosmetischen Kombinationsprodukts zur gleichzeitigen, getrennten oder zeitlich gestaffelten Verabreichung, enthaltend: eine erste kosmetische Zusammensetzung, die Glucosaminsulfat enthält, eine zweite kosmetische Zusammensetzung, die Hyaluronsäure enthält, und eine dritte kosmetische Zusammensetzung, die Kollagen zur Induktion der Hyaluronsäuresynthese durch Fibroblasten enthält.

2. Nicht-therapeutische kosmetische Verwendung nach Anspruch 1, wobei die zweite kosmetische Zusammensetzung, die Hyaluronsäure enthält, und die dritte kosmetische Zusammensetzung, die Kollagen enthält, dieselbe kosmetische Zusammensetzung bilden, in der die Hyaluronsäure und das Kollagen aus einem Knorpelextrakt stammen, vorzugsweise aus einem Knorpel, der aus einer Nahrungsquelle stammt, die von Fisch oder einem Säugetier, wie Schwein, Rind oder Geflügel, und besonders bevorzugt von einem Huhn, stammt.

3. Nichttherapeutische kosmetische Verwendung nach Anspruch 1, wobei die Hyaluronsäure der zweiten kosmetischen Zusammensetzung biotechnologischen und vorzugsweise bakteriellen Ursprungs ist.

4. Nicht-therapeutische kosmetische Verwendung nach einem der Ansprüche 1 bis 3, wobei das Kollagen durch ein Reinigungsverfahren aus Fisch, Säugetier oder Geflügel gewonnen wird.

5. Nicht-therapeutische kosmetische Verwendung nach einem beliebigen der Ansprüche 1 bis 4, wobei die Zusammensetzungen zur topischen Verabreichung bestimmt sind.

6. Nichttherapeutische kosmetische Verwendung nach einem beliebigen der Ansprüche 1 bis 4, wobei die Glucosaminsulfat enthaltende Zusammensetzung zur oralen Verabreichung und die Hyaluronsäure und/oder Kollagen enthaltenden Zusammensetzungen zur topischen Verabreichung bestimmt sind.

7. Nichttherapeutische kosmetische Verwendung nach einem beliebigen der Ansprüche 1 bis 4, wobei die Hyaluronsäure und/oder Kollagen enthaltende Zusammensetzung zur oralen Verabreichung und die Glucosaminsulfat enthaltende Zusammensetzung zur topischen Verabreichung bestimmt ist.

8. Nichttherapeutische kosmetische Verwendung nach einem beliebigen der Ansprüche 1 bis 4, wobei die Glucosaminsulfat enthaltende Zusammensetzung zur oralen Verabreichung bestimmt ist und die Hyaluronsäure und/oder Kollagen enthaltenden Zusammensetzungen kosmetische Füllerzusammensetzungen sind, die zur Verabreichung in die Haut bestimmt sind, insbesondere im Rahmen der Herstellung von Hyaluronsäurefüllern.

9. Nichttherapeutische kosmetische Verwendung nach einem beliebigen der Ansprüche 1 bis 4, wobei die Glucosaminsulfat und/oder Kollagen enthaltenden Zusammensetzungen zur oralen Verabreichung und die Hyaluronsäure enthaltende Zusammensetzung zur topischen Verabreichung bestimmt sind.

10. Nichttherapeutische kosmetische Verwendung nach einem der Ansprüche 1 bis 4, wobei die Glucosaminsulfat und/oder Kollagen enthaltenden Zusammensetzungen zur oralen Verabreichung bestimmt sind und die Hyaluronsäure enthaltende Zusammensetzung eine kosmetische Füllerzusammensetzung ist, die zur Verabreichung in die Haut bestimmt ist, insbesondere im Rahmen der Herstellung von Hyaluronsäurefüllern.

11. Nichttherapeutische kosmetische Verwendung nach einem beliebigen der Ansprüche 1 bis 10, wobei das Kombinationsprodukt mindestens 0,0001 Gew.-% Hyaluronsäure und/oder mindestens 0,1 Gew.-% Glucosaminsulfat und/oder mindestens 0,1 Gew.-% Kollagen, bezogen auf das Gesamtgewicht des kosmetischen Kombinationsprodukts, enthält.

12. Nichttherapeutische kosmetische Verwendung nach einem beliebigen der Ansprüche 1 bis 11, wobei das Kombinationsprodukt 10 bis 30 Gew.-% Hyaluronsäure, bezogen auf das Gesamtgewicht des kosmetischen Kombinationsprodukts, enthält.

13. Nicht-therapeutische kosmetische Verwendung nach einem beliebigen der Ansprüche 1 bis 12 zur Verstärkung des dermal-epidermalen Übergangsbereichs und/oder der Synthese von Hyaluronsäure durch die Dermis und das Bindegewebe des dermal-epidermalen Übergangsbereichs.

14. Nicht-therapeutische kosmetische Verwendung nach einem beliebigen der Ansprüche 1 bis 13 zur Bekämpfung von Alterserscheinungen, die mit dem Verlust von Binde- und Hautgewebe einhergehen.

15. Nicht-therapeutische kosmetische Verwendung nach einem beliebigen der Ansprüche 1 bis 13 zur Bekämpfung von Alterserscheinungen, die mit dem Verlust von Matrixgewebe, Trockenheit und papierartigem Aussehen der Haut einhergehen.

16. Nicht-therapeutische kosmetische Verwendung nach einem beliebigen der Ansprüche 1 bis 13 zur Bekämpfung von Alterserscheinungen, die mit dem Verlust von Matrixgewebe, dem Auftreten von Fältchen und Falten und dem Erschlaffen der Haut einhergehen.

17. Nichttherapeutische kosmetische Verwendung nach einem beliebigen der Ansprüche 1 bis 13 zur Bekämpfung der Hautalterung, die die Epidermis betrifft, insbesondere zur Bekämpfung von Störungen der epidermalen Hydratation und Schuppenbildung, vor allem von solchen, die durch intensive Sonneneinstrahlung verschlimmert werden.

18. Nicht-therapeutische kosmetische Verwendung nach einem beliebigen der Ansprüche 1 bis 13 zur Verbesserung der Wirkung und Dauer von kosmetischen Behandlungen auf Hyaluronsäurebasis, insbesondere der Wirkung von Hyaluronsäure enthaltenden Füllerzusammensetzungen.

19. Nicht-therapeutische kosmetische Verwendung nach einem beliebigen der Ansprüche 1 bis 13 zur Verstärkung der Hydratation der Haut.

20. Nichttherapeutische kosmetische Verwendung nach einem beliebigen der Ansprüche 1 bis 13 zur Herstellung von rekonstruierter Haut und Hautimplantaten.

21. Nicht-therapeutisches kosmetisches Verfahren zur Induktion der Hyaluronsäuresynthese durch Fibroblasten, wobei ein kosmetisches Kombinationsprodukt gemäß einem beliebigen der Ansprüche 1 bis 12 verabreicht wird.

22. Nichttherapeutisches kosmetisches Verfahren nach Anspruch 21 zur Begrenzung des Auftretens von Falten, Fältchen und Erschlaffung des Gesichts.

23. Nicht-therapeutisches kosmetisches Verfahren nach Anspruch 21 zur Bekämpfung des Wiederauftretens von Falten und Erschlaffung des Gesichts nach Injektionen von Hyaluronsäure in die Haut, im Gesicht und/oder an der Gesamtheit der Körperteile, die Gegenstand eines nachfolgenden oder begleitenden restaurativen, ästhetischen oder kosmetischen Injektionsverfahrens waren, ohne jedoch 6 Monate zwischen den beiden Verfahren zu überschreiten.

24. Nicht-therapeutisches kosmetisches Verfahren nach Anspruch 21 zur Verlängerung der Wirkungsdauer von ästhetischen Behandlungen, die mit Hilfe von Füllerzusammensetzungen, insbesondere auf der Basis von Hyaluronsäure, durchgeführt werden, die in die Haut zum Auffüllen des Gesichts, des Körpers und der Falten injiziert werden.

## Claims

1. Cosmetic non therapeutic use of a cosmetic combination product for an administration that is simultaneous, separated or spread over time, comprising: a first cosmetic composition comprising glucosamine sulfate, a second cosmetic composition comprising hyaluronic acid and a third cosmetic composition comprising collagen to induce the acid hyaluronic synthesis by the fibroblasts.

2. Cosmetic non therapeutic use according to claim 1, wherein said second cosmetic composition comprising hyaluronic acid and said third cosmetic composition comprising collagen constitute one cosmetic composition wherein the hyaluronic acid and collagen are derived from a cartilage extract, preferably a cartilage from a food source from fish or mammal such as pork, beef or poultry, and more preferably from chicken.

3. Cosmetic non therapeutic use according to claim 1, wherein the hyaluronic acid of the second cosmetic composition is of biotechnological origin and of preferably bacterial.

4. Cosmetic non therapeutic use according to any of claims 1 to 3, wherein the collagen is derived from a purification method from fish, mammal or poultry.

5. Cosmetic non therapeutic use according to any one of claims 1 to 4, wherein said compositions are to be administered topically.

6. Cosmetic non therapeutic use according to any one of claims 1 to 4, wherein the composition comprising glucosaminesulfate is to be administered orally and the compositions comprising hyaluronic acid and/or collagen are intended to be administered topically.

7. Cosmetic non therapeutic use according to one of claims 1 to 4 wherein the composition comprising hyaluronic acid and/or collagen are intended to be administered by the oral route and the composition comprising glucosamine sulfate is to be administered topically.

8. Cosmetic non therapeutic use according to one of claims 1 to 4, wherein the composition comprising glucosamine sulfate is to be administered orally and the compositions comprising hyaluronic acid and/or collagen compositions are cosmetic fillers (filler) to be administered into the skin, in particular in the context of the preparation of fillers with hyaluronic acid.

9. Cosmetic non therapeutic use according to one of claims 1 to 4, wherein the compositions comprising glucosamine sulfate and/or collagen are intended to be administered orally and the composition comprising hyaluronic acid is to be administered topically.

10. Cosmetic non therapeutic use according to any one of claims 1 to 4, wherein the compositions comprising glucosamine sulfate and/or collagen are intended to be administered orally and wherein the composition comprising hyaluronic acid is a cosmetic composition fillers (filler) to be administered into the skin, in particular in the context of the preparation of fillers with hyaluronic acid.

11. Cosmetic non therapeutic use according to one of claims 1 to 10, wherein the combination product comprising at least 0.0001% hyaluronic acid and/or at least 0.1% glucosamine sulfate and/or at least 0.1% of collagen, by weight relative to the total weight of the cosmetic combination product.

12. Cosmetic non therapeutic use according to one of claims 1 to 11, wherein the combination product comprising 10 to 30% hyaluronic acid by weight relative to the total weight of the cosmetic combination product.

13. Cosmetic non therapeutic use according to any one of claims 1 to 12, to enhance the dermal epidermal junction and/or the synthesis of hyaluronic acid in the dermis and connective tissue of the dermal-epidermal junction.

14. Cosmetic non therapeutic use according to any one of claims 1 to 13 to combat the signs of aging linked to the loss of connective tissue and skin.

15. Cosmetic non therapeutic use according to any one of claims 1 to 13 to combat the signs of aging linked to the loss of matrix tissue, dryness and dry and thin appearance of the skin.

16. Cosmetic non therapeutic use according to any one of claims 1 to 13, to combat the signs of aging linked to the loss of matrix tissue, the appearance of lines and wrinkles and sagging skin.

17. Cosmetic non therapeutic use according to any one of claims 1 to 13, to combat skin aging affecting the skin, in particular to combat the disorders of hydration and flaking of the skin, in particular those aggravated by intense sun exposure.

18. Cosmetic non therapeutic use according to any one of claims 1 to 13, to enhance the effects and duration of cosmetic treatment based on hyaluronic acid, particularly the effect of filler compositions (filler) comprising hyaluronic acid.

19. Cosmetic non therapeutic use according to any one of claims 1 to 13, to enhance skin hydration.

20. Cosmetic non therapeutic use according to any one of claims 1 to 13 for the preparation of reconstructed skin and subcutaneous implants.

21. Cosmetic non therapeutic method for inducing the synthesis of hyaluronic acid, by the fibroblasts, wherein a cosmetic combination product as defined in any one of claims 1 to 12 is administered.

22. Cosmetic non therapeutic method according to claim 21 for limiting the appearance of fine lines, wrinkles and facial sagging.

23. Cosmetic non therapeutic method according to claim 21, to combat the reappearance of wrinkles and facial sagging after injections of hyaluronic acid in the skin on the face and/or on all body parts that have been the subject of a restorative injection procedure, aesthetic or cosmetic subsequent or concurrent, but not exceeding six months between the 2 procedures.

24. Cosmetic non therapeutic method according to claim 21 for extending the duration of efficacy of beauty treatments made using filler compositions (filler), in particular based on hyaluronic acid injected into the skin to bridging the face, body and wrinkles.
